# EUROPEAN PATENT APPLICATION

(11) **EP 1 908 821 A1**
(43) Date of publication of application: **09.04.2008**
(21) Application number: 06121795.6
(22) Date of filing: 05.10.2006
(51) Int. Cl.: C12N 5/06, C12N 15/863, A61K 39/285

(54) **Use of mva as adjuvant in the stimulation of antigen-presenting cells**

(71) Applicant: Helmholtz-Zentrum für Infektionsforschung, 38124 Braunschweig (DE); GSF-Forschungszentrum für Umwelt und Gesundheit GmbH, 80807 München (DE)
(72) Inventor: Guzmàn, Carlos, 38304 Wolfenbüttel (DE); Becker, Pablo Daniel, 38300 Wolfenbüttel (DE); Nörder, Miriam, 27753 Delmenhorst (DE); Drexler, Ingo, 80797 München (DE); Erfle, Volker, 81679 München (DE)
(74) Representative: Behnisch, Werner

(57) **Abstract**

The present invention is directed to a method for the activation/maturation of antigen presenting cells (APC's). The present invention further is directed to a pharmaceutical composition containing same and to the use of non-recombinant MVA as an adjuvant for the stimulation of APC's.

## Description

The present invention is directed to a method for the activation/maturation of antigen presenting cells (APC's). The present invention further is directed to a pharmaceutical composition containing same and to the use of non-recombinant MVA as an adjuvant for the stimulation of APC.

### Background of the invention

In 1953, German scientists obtained the vaccinia virus Ankara strain, which was originally obtained at the Turkish institute in Ankara by passages alternating between donkeys and calves [58]. The virus strain was established as chorioallantois vaccinia virus Ankara (CVA), because it was routinely cultivated and titered on the chorioallantois membrane of embryonated chicken eggs. Some years later, A. Mayr reported that CVA had developed attenuated growth characteristics on the chorioallantois membrane, in tissue cultures and in laboratory animals after 371 passages on chicken embryo fibroblasts (CEFs) in the Bavarian State Vaccine Institute [59,60]. Upon further passages the phenotype was stabilised, and the 516^{th} passage virus was renamed as modified vaccinia virus Ankara [52]. As consequence of these passages the virus was noted to be less virulent than its parent strain. From 1968, MVA vaccine has been safely administered to more than 100,000 people in Germany, including many persons considered at risk for conventional vaccination (e.g., immunocompromised, elderly) without documentation of any complication [59,61,62]. Today, MVA vectors are handled in USA and Germany under biosafety level 1 conditions [63,64].

Recently, the complete genome of MVA has been sequenced (from an MVA preparation provided as standard obtained in the 572^{nd} CEF passage), and the genes lost during attenuation have been identified [65]. During attenuation, MVA lost ~15% of its CVA parent genome (ca. 30 kb) including genes for virus host range regulation and evasion of the host immune response [52]. Thus, MVA replication is extremely limited in mammalian cells [52], being blocked at a very late stage of the virus life cycle with no alteration in early or late virus gene expression or in the levels of expression of heterologous proteins [66]. It was demonstrated that the immune responses stimulated against a foreign MVA-encoded antigen were at least as high, or even higher at certain viral doses, than those stimulated by a replication competent strain. Early events in the life cycle and/or advantages in transcription or translation due to multiple deletions in functional genes could account for this enhancement. The more efficient foreign gene expression is cell type independent [67]. Furthermore, several natural deletion sites can be found in the genome of MVA distributed in clusters, which can serve as insertion sites for foreign genes without affecting MVA gene functions. The deletions II, III and VI have been preferentially used because they are located in the more conserved regions of the genome, therefore, they are not affected by sequence rearrangements. The viral thymidine kinase is the standard insertion locus used in recombinant vaccinia viruses and it has been also used to obtain recombinant MVA [56]. The integration of a foreign gene sequence into the MVA genome can be directed by homologous recombination using transfer plasmids that contain MVA DNA fragments homologous to DNA sequences flanking the targeted insertion site. In addition, the K1L gene is not expressed in MVA, causing an early block of viral replication upon infection of rabbit kidney RK-13 cells. However, when MVA is complemented, the defective virus is able to grow in these cells. Thus, a method to obtain recombinant MVA was developed, in which the transient introduction of the K1L gene into the viral genome can be used as a selectable growth marker [57,63,68].

An important issue to be considered in the context of using MVA as a vector is the role played by pre-existing immunity against vaccinia virus in a large part of the human population [69]. It has been suggested that, if recombinant poxviruses are applied to humans, these individuals can not receive further vaccines based on the same vectors. However, it was shown that MVA is less affected by pre-existing vaccinia-specific immunity than the replication competent Western Reserve (WR) strain [69]. Weak anti-virus immune responses were triggered by MVA at both humoral and cellular levels [67]. However, the immune responses against the foreign antigen were nearly fourfold higher than those observed in mice pre-immunised with the WR strain [69]. The potential problem of impaired effectiveness of the MVA vector because of pre-existing immunity can be overcome by different strategies. The use of either different routes for priming and boosting or the combination with DNA vaccines could provide an answer [57]. It was demonstrated that after subcutaneous vaccination with vaccinia, the inductive sites of the mucosal immune system may still be naive to the vaccinia antigens. Therefore, immunisation by the mucosal route can be used for the induction of recombinant protein-specific responses in individuals with pre-existing immunity to the carrier [70].

Recently, recombinant MVA expressing immunogens from a variety of infectious agents (e.g., human immunodeficiency virus [HIV], human papilloma virus [HPV], *Plasmodium falciparum*) or tumour-associated antigens (e.g., melanoma) have reached phase I and II clinical trials [50,57,71]. Furthermore, the ability of MVA vaccines to amplify pre-existing T cell responses induced by priming with DNA was shown in animal models. For example, the implementation of this approach resulted in high levels of CTLs in macaques, as well as memory responses against multiple simian immunodeficiency virus (SIV) and HIV antigens [57].

Interestingly, there is a discrepancy between the excellent results observed using recombinant MVA *in vivo,* and *in vitro* studies showing that MVA can impair the viability and functions of DCs. The fact that MVA, being a virus, could exhibit *per se* immune modulatory properties has not been satisfactorily addressed up to now.

### Summary of the invention

Therefore, it is an object of the invention to provide an adjuvant for improving the immunogenicity of antigens in an attempt to activate/mature antigen presenting cells (APC) *ex vivo.* It is a further object of the invention to provide a means for increasing the natural immune response to one or more antigens in a patient.

These objects are achieved by the subject-matter of the independent claims. Preferred embodiments are set forth in the dependent claims.

According to the invention, the above objects are achieved by using MVA, preferably non-recombinant MVA, as an adjuvant in the activation/maturation of APC's.

DCs are the most potent APCs of the immune system and play a key role in the initiation, maintenance and fine-tuning of adaptive immune responses [25,82]. The cellular production of molecules of the TNF family and type I IFN represents a first line of immune defence following virus infection, which can lead to the apoptosis of virus-infected cells and/or inhibition of viral replication. In this context, several viruses (e.g., vaccinia) have developed immune escape mechanisms leading to an affected DC maturation, which in turn leads to an impaired presentation of viral antigens. This can be achieved by the expression of molecules that inhibit secretion or function of those cytokines promoting DC maturation (e.g., TNF, IL-1). However, MVA has lost most of these immune evasion genes during its attenuation in CEFs [65,87]. Thus, it is extremely difficult to conciliate the excellent performance of MVA as vaccine vector with the data generated by Behboudi *et al.* suggesting that the infection of iDCs with MVA results in the down-regulation of MHC class I molecules and impaired capacity to stimulate T cells [82].

Two different hypotheses were suggested to explain these observations. On the one hand, apoptotic cells could be taken up, processed and presented by DCs to prime and expand CTL *in vivo* [88]. However, antigens delivered to DCs via apoptotic cells mostly fail to activate DCs and may even promote tolerance *in vivo* [89-92]. Nevertheless, one can assume that the ability of DCs to cross-present antigens from apoptotic cells may be dependent on the specific microenvironment [88,93]. On the other hand, it was proposed that MVA particles bound to DC that were not removed by washes could be released after DC vaccination and became potent inducers of CTL responses. However, this second explanation seems unlikely, due to the low number of attached viral particles (<10⁴ p.f.u.) [82].

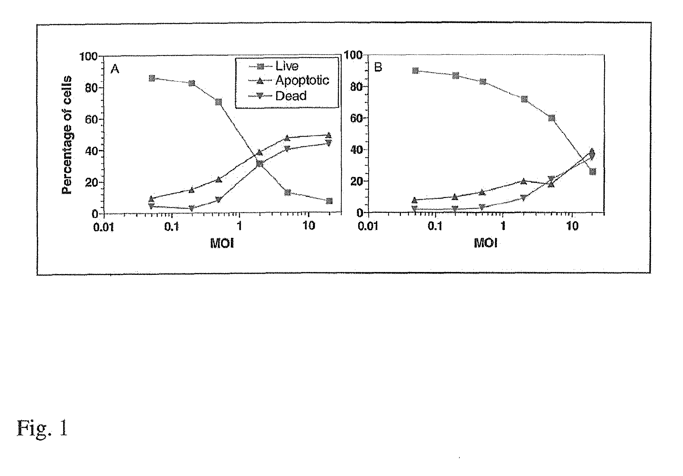

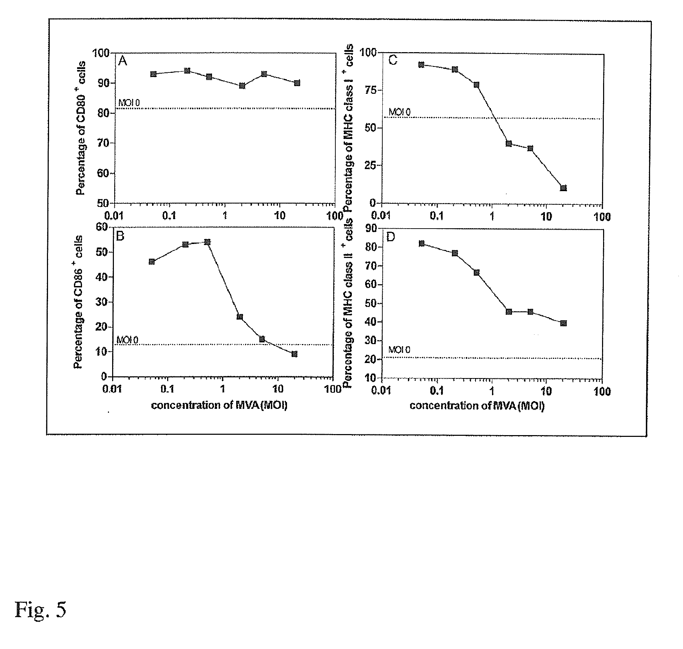

The experimental hypothesis in this invention was that the reported results were, at least in part, an artefact resulting from suboptimal infection conditions for the *in vitro* studies. To address this issue, a dose-response study was performed in which DCs were infected with MVA at different MOI using the same conditions described by this group. The obtained results demonstrated that, as reported, infection at high MOI resulted in dysfunction and apoptosis of DCs. However, the use of low MOI (0.02 to 0.5) resulted in an enhanced activation and maturation of DCs, which also exhibit improved antigen presentation functions. Washed DCs even showed a stronger up-regulation at low MOIs (Fig. 5). In contrast, the activation and maturation of DCs infected with MVA at a MOI ≥ 5 was similar or even lower than that observed in control uninfected cells. Furthermore, a dose-dependent increment in the numbers of apoptotic cells was observed in DCs infected with MVA at MOI ≥ 5, whereas at lower MOI the viability of DCs was preserved (Fig. 1).

Vaccination with purified antigens is often insufficient to stimulate strong immune responses, hence, adjuvants are required. In this context, the immune system has evolved to respond to danger situations, such as infections. Therefore, molecules delivering "danger" signals are currently investigated for their potential as adjuvants [94]. Immune responses can be enhanced by the production of inflammatory cytokines, which in turn can stimulate DCs leading to an improved antigen processing and presentation. As attenuated virus, MVA possesses the ideal profile to be investigated as adjuvant. *In vitro* studies in this work have demonstrated that DC infection with MVA at low MOI resulted in a strong stimulation of their activation and maturation. All the investigated activation markers were upregulated, and infected DCs exhibited an enhanced capacity to process and present epitopes to both CD4⁺ and CD8⁺ T cells. On the other hand, the *in vivo* studies showed that co-administration of MVA resulted in consistently enhanced immune responses. This represents a real breakthrough, since it would be possible to add-mix purified antigens with MVA, rather than developing a genetically modified MVA producing it.

Surprisingly, an important T_{H}2 component was observed when MVA was used as adjuvant, with strong antibody responses characterized by high IgG1 titres. This is in contrast to what was observed using recombinant MVA, which are characterized by strong CTL responses, the stimulation of low antibody titres and a T_{H}1 dominant response pattern [67]. Thus, it might be possible to modulate the elicited responses depending on the fact that antigens are either co-administered with MVA or expressed by recombinant viruses. The effect of MVA as adjuvant may base on its ability to activate DCs, followed by the stimulated secretion of pro-inflammatory cytokines. Antigen uptake, processing and presentation by DCs also seems to be enhanced in the presence of MVA.

Therefore, it was shown for the first time in the present invention that MVA (preferably, non-recombinant MVA) may be used as an effective adjuvant in the maturation/stimulation of APC's.

### Detailed description of the invention

The present invention is directed to the following aspects and embodiments:

In a first aspect, the present invention provides a method for *ex vivo* stimulation of antigen presenting cells (APC) comprising the steps of:
a) providing APC's and a composition containing one or more antigens and modified vaccinia virus Ankara (MVA) as an adjuvant;
b) infecting the APC's with the composition such that they are infected by MVA at a low MOI, thereby activating and maturing the APC's; and
c) isolating the activated/mature APC's.

The MVA used in this invention preferably are non-recombinant MVA. It is noted that, in the present invention, the term "non-recombinant" means MVA which does not contain foreign sequences, i.e. sequences which are expressed via that MVA.

According to the present knowledge, the expert would assume that co-incubation with MVA of APC (more specifically DC) would lead to apoptosis. However, dead cells would perhaps be disadvantageous for a cellular vaccine. Furthermore, due to intrinsic variability the standardization of a GMP process would be extremely difficult. The present data demonstrate that, under optimal conditions presented herein, this is not the case, and that the process of activation and maturation is optimized, thereby making the APC's more efficient in terms of taking up, processing and presenting to specific T cells the tested antigens.

Preferably, the MOI is between 0.02 and 0.5, preferably 0.05. As mentioned above, the use of low MOI (0.02 to 0.5) resulted in an enhanced activation and maturation of DCs, which also exhibit improved antigen presentation functions.

The APC preferably is selected from dendritic cells, activated B cells, monocytes, macrophages, activated T cells, hematological malignancies with antigen presenting capacities, EBV-transformed lymphoblastoid cell lines and/or lymph or vascular endothelial cells. Most preferably, they are dendritic cells (DC).

The antigens contained in the above composition are not restricted in their kind and may be deliberately selected from any substance, which might be useful to create / enhance an immune response *in vivo.* Thus, an antigen for the purposes of the present invention may be defined as a substance that stimulates an immune response. Antigens as defined herein are usually proteins or polysaccharides, but can be any type of molecule, including small molecules (haptens) coupled to a carrier-protein.

The term "antigen" comprises purified as well as other materials having antigenic properties, for example inactivated agents (e.g. whole Bordetella pertussis). The term encompasses also mixtures of two or more agents, and additionally of an antigen with any other vaccine and adjuvant.

The antigen may preferably be derived from the group consisting of therapeutic or prophylactic polypeptides, glycoproteins, lipoproteins, or polysaccharides and polypeptides, glycoproteins, lipoproteins, or polysaccharides of pathogenic agents, and functional parts thereof.The term "functional part thereof" in this context means that this part is still sufficient in order to show its function as an antigen in the meaning of the above presented definition.

In an embodiment, the therapeutic polypeptide is derived from the group consisting of secreted proteins, e.g. polypeptides of antibodies, chemokines, cytokines or interferons.

Pathogenic agents are to be understood to be viruses, bacteria, fungi, protozoa and parasites which may cause a disease, as well as tumor cells which multiply unrestrictedly in an organism and may thus lead to pathological growths, and functional parts thereof.

Examples of such pathogenic agents are described in Davis, B.D. et al., (Microbiology, 3rd ed., Harper International Edition). Preferred genes of pathogenic agents are those of influenza viruses, of measles and respiratory syncytial viruses, of dengue viruses, of human immunodeficiency viruses, for example HIV I and HIV II, of human hepatitis viruses, e.g. HCV and HBV, of herpes viruses, of papilloma viruses, of the malaria parasite Plasmodium falciparum, and of the tuberculosis-causing Mycobacteria.

Preferred genes encoding tumor associated antigens are those of melanoma-associated differentiation antigens, e.g. tyrosinase, tyrosinase-related proteins 1 and 2, of cancer testes antigens, e.g. MAGE-1,-2,-3, and BAGE, of non-mutated shared antigens overexpressed on tumors, e.g. Her-2/neu, MUC-1, and p53.

If recombinant MVA is used in the practice of the present invention, it may encode one or more of the above mentioned antigens.

According to a second aspect, the present invention provides a pharmaceutical composition containing the composition as defined above or a pharmaceutically acceptable carrier and/or diluent.

The term "pharmaceutically acceptable" is defined as meaning a non-toxic material, which does not interfere with effectiveness of the biological activity of the active component. The choice of the carrier is dependent on the application.

The pharmaceutical composition can contain additional components which enhance the activity of the active component or which supplement the treatment. Such additional components and/or factors can be part of the pharmaceutical composition to achieve synergistic effects or to minimize adverse or unwanted effects.

Furthermore, such a pharmaceutical composition may contain further adjuvants, which are known to increase/modulate immune responses in a patient. As examples of such adjuvants, one can name compounds with immunomodulatory properties, preferably cytokines, chemokines, adjuvants/ immunomodulators, e.g., TLR-agonists such as MALP-2, poly IC, CpG, LPS, MPL.

For further information, it is referred to Weigt H, Muhlradt PF, Larbig M, Krug N, Braun A.: The Toll-like receptor-2/6 agonist macrophage-activating lipopeptide-2 cooperates with IFN-gamma to reverse the Th2 skew in an in vitro allergy model. J Immunol. 2004 May 15;172(10):6080-6.

Techniques for the formulation or preparation and application/medication of compounds of the present invention are published in "Remington's Pharmaceutical Sciences", Mack Publishing Co., Easton, PA, latest edition.

The above activated/mature APC's are contained in the pharmaceutical preparation in a therapeutically effective dose. A therapeutically effective dose relates to the amount of a substance which is sufficient to improve the symptoms, for example a treatment, healing, prevention or improvement of such conditions. An appropriate application can include for example parenteral application, including intramuscular, subcutaneous, intramedular injections, as well as intrathecal, direct intraventricular, intravenous or intraperitoneal injections. The intravenous injection is the preferred treatment of a patient.

The pharmaceutical composition can be used for prophylactic and/or therapeutic treatments.

In a preferred embodiment, the pharmaceutical composition is a vaccine and may additionally comprise other compounds with immunomodulatory properties, preferably cytokines, chemokines, adjuvants/ immunomodulators, e.g., TLR-agonists such as MALP-2, poly IC, CpG, LPS, MPL (see above).

In a third aspect, the invention provides the use of MVA, preferably non-recombinant MVA, as an adjuvant in the stimulation of APC's *ex vivo.*

In a fourth aspect, the use of the pharmaceutical composition of the invention in the manufacture of a medicament for the treatment of infectious diseases, chronic inflammatory or degenerative diseases, autoimmunity diseases, cancer, fungal diseases and allergies is disclosed. Basically, all diseases of chronic or acute, infectious and non-infectious type which might be prevented or treated.

The present invention is further illustrated in the following by the non-limiting examples and the figures.

In the figures, the following is depicted:

Fig.1 Analysis of DCs viability after infection with MVA. Primary cultures of BM-DCs were prepared as described in the Methods section, and a comparative analysis of the viability of washed (A) and unwashed (B) cells was performed after infection with MVA by flow cytometry. The number of apoptotic (Annexin V-positive) and dead (7-AAD-positive) cells was determined by gating on CD11c positive cells.

Fig. 2 Comparison between cell viability of washed and unwashed uninfected DC.

Fig. 3 DCs viability after infection with MVA. Cells were infected for 6 h, washed and further incubated for 16 h. The number of apoptotic (Annexin V-positive) and dead (7-AAD-positive) cells was determined by FACS gating on CD11c positive cells.

Fig. 4 Efficient T-cell activation depends on both signal 1 and 2. Professional APCs, such as DCs, provide more-effective co-stimulation (signal 2) and, therefore, are able to efficiently activate T cells.

Fig. 5 Expression of activation markers by washed DCs infected with MVA. Cells labelled with FITC conjugated antibodies against CD80 (A), CD86 (B), MHC class I (C) and MHC class II (D) were analysed by FACS, gating on CD11c⁺ cells.

Fig. 6 DC activation by MVA infection. Washed DCs were mock-infected with (i) medium (MOI=0, (ii) with 0.05 or (iii) with 5 p.f.u. per cell. Cells labelled with anti-CD86-FITC and anti-MHC class I-FITC antibodies were analysed by flow cytometry, gating on CD11c+ cells.

Fig. 7 Effect of MVA as adjuvant on the viability of infected DC in the presence or absence of Ova. Primary cultures of DCs were prepared as described in the Methods section, and an analysis of the viability of cells was performed after infection with MVA. The number of apoptotic (Annexin V-positive) and dead (7-AAD-positive) cells was determined by FACS gating on CD11c positive cells.

Fig. 8 Expression of activation and maturation makers by DCs infected with MVA in the presence or absence of the model antigen Ova. Cells labelled with FITC conjugated antibodies against CD80, CD86, MHC class I and MHC class II were analysed by FACS, gating on CD11c⁺ cells. (A) Histograms of CD86 expression on DCs after infection in absence or presence of Ova at different MOIs . (B) Analysis of the expression markers on the DC surface at different MOIs in comparison to the expression of DCs incubated with Ova alone.

Fig. 9 Proliferation of T cells from OT-II mice after incubation with uninfected or MVA-infected DCs in absence (A) or presence (B) of Ova. Either DCs infected with the peptide (ISQAVHAAHAEINEAGR) or with the protein (Ova) were used as controls.

Fig. 10 Proliferation of T cells from OT-I mice after incubation with uninfected or MVA-infected DCs in absence (A) or presence (B) of Ova. Either DCs infected with the peptide (SIINFEKL) or with the protein (Ova) were used as controls.

Fig. 11. Weights of immunised mice (means per group).

Fig.12 Production of anti-Ova serum IgG in mice vaccinated using MVA as adjuvant. Each bar represents the group mean end-point titre of anti-Ova IgG. The standard error of the mean is indicated by vertical lines. The results were statistically significant when compared with ANOVA at p<0.001 (***).

Fig. 13 Anti-Ova serum IgG1/IgG2c-titres obtained in mice vaccinated using MVA as adjuvant. Each bar represents the group mean end-point titre. The standard error of the mean is indicated by vertical lines.

Fig. 14 Numbers of IL-2- and IL-4-producing cells in mice vaccinated using MVA as adjuvant. Cells were re-stimulated with Ova protein. Results are presented as specific IL-4-(left panel) and IL-2-spot forming units (SFU)/5 x 10⁵ cells (right panel). The standard error of the mean of quadruplicate values is indicated by vertical lines. The values reported are those obtained from stimulated cells subtracted of background from unstimulated cells. Unstimulated cells were lower than 30 SFU/5 x 10⁵ cells. The results were statistically significant when compared with ANOVA at p<0.05 (*).

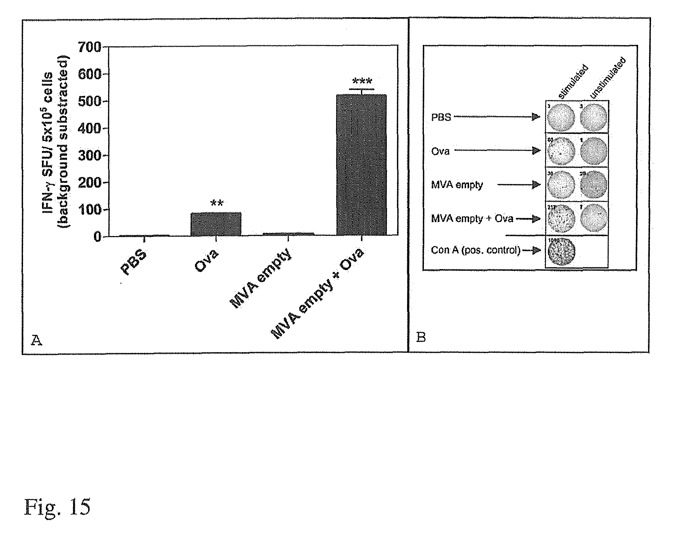

Fig. 15 Numbers of IFN-γ-producing CD8⁺ cells in mice vaccinated using MVA as adjuvant. (A) Cells were re-stimulated with the dominant Ova-peptide SIINFEKL. Results are presented as specific IFN-γ spot forming units (SFU)/5 x 10⁵ cells. The standard error of the mean of quadruplicate values is indicated by vertical lines. The values reported are those obtained from stimulated cells subtracted of background from unstimulated cells. Unstimulated cells were lower than 30 IFN-γ- SFU/5 x 10⁵ cells. (B) Every spot is representing a single IFN-γ secreting cell. The results were statistically significant when compared with ANOVA at p<0.01 (**) and p<0.001 (***).

Fig. 16 In vivo analysis of specific lysis stimulated in mice vaccinated with MVA as adjuvant. Labelled control (CFSEI^{lo}) and target cells pulsed with the Ova-peptide SIINFEKL (CFSE^{hi}) were transferred to vaccinated mice. After 40 h mice were sacrificed and in vivo lysis was analysed by FACS. Each bar represents the group mean for the % of specific lysis.

### Examples:

### 1. Characterisation of the effect of MVA on the activation, maturation and biological functions of DCs

DCs are professional APCs with the capacity to activate CD4⁺ and CD8⁺ T lymphocytes. They are able to degrade virus-derived antigens into peptides, which are then displayed on the cellular surface in complex with MHC molecules. The access to DCs *in vivo* may be critical for MVA and other poxviruses in order to induce potent immune responses [81]. Thus, it is extremely difficult to conciliate the excellent *in vivo* performance of MVA as vaccine vector with the *in vitro* data generated by Behboudi *et al*., which suggested that infection of DCs with MVA triggered cellular apoptosis [81,82]. In addition, infected DCs were dysfunctional and expressed low levels of MHC class I molecules. Thus, in this work an alternative hypothesis was evaluated that might explain the previous results, namely that they represent an artefact resulting from the selection of suboptimal infection conditions for the *in vitro* studies. To address this issue, a dose-response study was performed in which DCs were infected with MVA at different MOI using the same conditions described by this group. As mentioned in the methods section, the stimulation of isolated DCs was performed in two different ways (Fig. 1). DCs were stimulated with different concentrations of MVA (MOI 0.05-20) for:
1. 16 h without washes, or
2. 6 h, followed by three washes with PBS to remove unbound MVA and further incubation for 16 h.

### 1.1 Viability of DCs following MVA infection

The number of CD11c positive cells after 6 days of culture was approximately 75 and 60% in washed and unwashed DC preparations, respectively. To determine whether MVA induces apoptosis/necrosis in infected DCs, cell viability was analysed by flow cytometry using Annexin V and 7-AAD. Thus, it was possible to discriminate between live, dead and apoptotic cells.

Fig.1: Analysis of DCs viability after infection with MVA. Primary cultures of BM-DCs were prepared as described in the Methods section, and a comparative analysis of the viability of washed (A) and unwashed (B) cells was performed after infection with MVA by flow cytometry. The number of apoptotic (Annexin V-positive) and dead (7-AAD-positive) cells was determined by gating on CD11c positive cells.

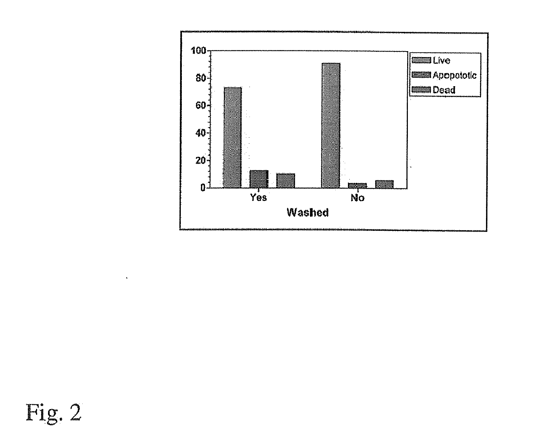

Infection of DCs with high MOI of MVA led to increased numbers of dead and apoptotic cells (Fig. 1). More than half of the DCs stimulated with the highest concentration of MVA (MOI 20) were apoptotic or dead. In contrast, approximately 80% of the cells infected at low MOI (0.05-0.2) retained their viability (Fig. 1). The number of live cells decreased in both, washed and unwashed, preparations in a dose-dependent manner. The maximum number of dead/apoptotic cells was observed at MOI 20. Interestingly, the amount of live cells was reduced by washing the cells three times with PBS. After these washes, about 75% of non-infected cells were alive, whereas without washes about 90% of non-infected cells were alive (Fig. 2). This suggests that the mechanical stress led to a consistent reduction in cell viability. Fig. 2: Comparison between cell viability of washed and unwashed uninfected DC.

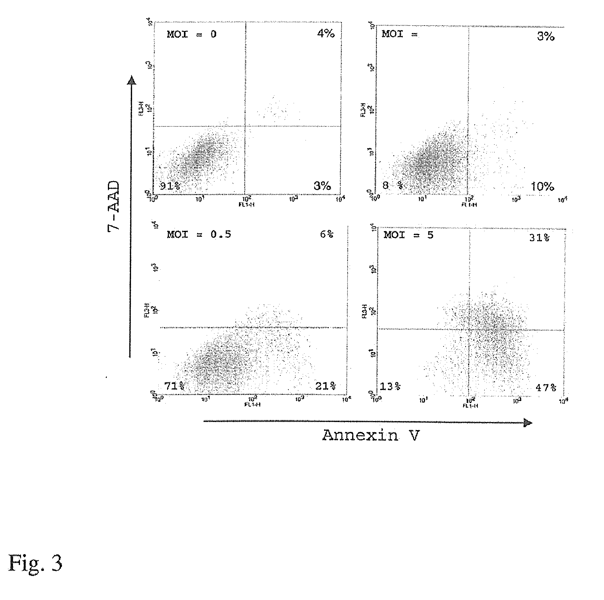

It has been demonstrated that cell survival at low MOI was similar in washed and unwashed DCs. In contrast, infection at a MOI of 5, as described by Behboudi et al. [82] induced a high number of dead cells in washed DCs (Fig. 3).

Fig. 3: DCs viability after infection with MVA. Cells were infected for 6 h, washed and further incubated for 16 h. The number of apoptotic (Annexin V-positive) and dead (7-AAD-positive) cells was determined by FACS gating on CD11c positive cells.

### 1.2 Expression of activation and maturation markers by DCs after infection with MVA

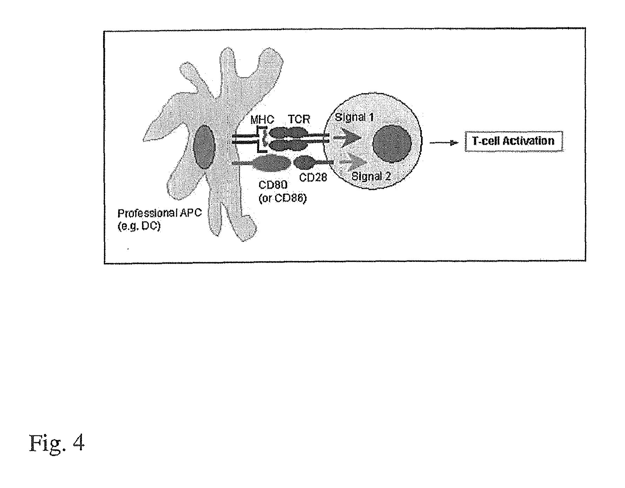

To evaluate the effect of MVA infection on DC activation, a phenotypic analysis for the expression of MHC class I and II, co-stimulatory (CD80 and CD86) and adhesion (CD40) molecules by FACS was performed. CD80 is an important activation marker, however, it is also expressed in basal levels on immature dendritic cells (iDCs) [83]. On the other hand, CD86 is a sensitive activation marker in the murine system [83]. These two molecules are essential for the capacity of DCs to stimulate T cells. In fact, the presentation of processed peptides in the context of MHC molecules (signal 1) without co-stimulation (signal 2) is insufficient for T cell activation, and can even lead to the induction of tolerance (Fig. 4) [84]. Fig. 4: Efficient T-cell activation depends on both signal 1 and 2. Professional APCs, such as DCs, provide more-effective co-stimulation (signal 2) and, therefore, are able to efficiently activate T cells.

When DCs were infected with MVA at low MOI, an up-regulated expression of activation markers under both experimental conditions was observed (*i.e.*, washed [Fig. 5], unwashed [data not shown]). In contrast, at high MOI the corresponding markers were expressed at lower levels than in untreated cells (with exception of CD80 expression).
Fig. 5: Expression of activation markers by washed DCs infected with MVA. Cells labelled with FITC conjugated antibodies against CD80 (A), CD86 (B), MHC class I (C) and MHC class II (D) were analysed by FACS, gating on CD11c⁺ cells.

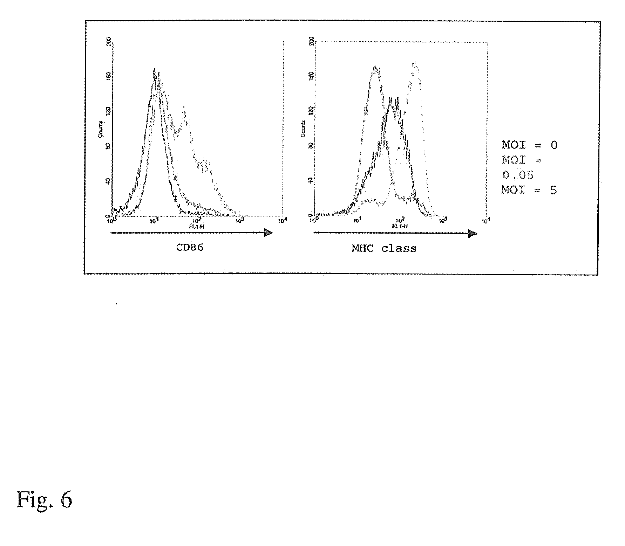

DCs showed an up-regulated expression of CD86 at low MOI (0.02-0.5) and no activation or even a down-regulation at high MOIs (Fig. 5). CD80 was also up-regulated when DCs were infected with MVA, but the activation was not dependent of the MOI. MHC class I was expressed at high levels at low MOI, and the expression was MOI-dependent, showing a down-regulation at high MOIs. MHC class II showed an up-regulation at high and low MOIs. However, DCs showed a dose dependent regulation with a higher expression at low MOIs (~80%) than at high MOIs (~40%) (Fig. 5). In conclusion, the expression of CD80 and MHC class II molecules was up-regulated at all tested MOI (0.05-20). In contrast, a dose-dependent expression of CD86 and MHC class I molecules was observed (Fig. 6).

Fig. 6 DC activation by MVA infection. Washed DCs were mock-infected with (i) medium (MOI=0, (ii) with 0.05 or (iii) with 5 p.f.u. per cell. Cells labelled with anti-CD86-FITC and anti-MHC class I-FITC antibodies were analysed by flow cytometry, gating on CD11c+ cells.

### 2. Exploitation of MVA as an adjuvant for vaccine design

The above presented results demonstrated that MVA exerts potent immunomodulatory activities on DCs *ex vivo.* Thus, it was investigated if these biological properties can also be exploited for optimizing the immune responses stimulated by soluble antigens. To this aim, *in vitro* and *in vivo* studies using Ova as a model antigen and wild type empty MVA as adjuvant were performed.

### 2.1 In vitro evaluation of MVA as immunomodulator

To assess the effect of MVA on DCs, cells were infected in the presence or absence of the model antigen Ova at different MOI (*i.e.,* 0.05, 0.5 and 5) for 6 h. Then, cells were washed three times with PBS to remove unbound virus, further incubated for 16 h and analysed.

### 2.1.1 Effect on the DC viability using MVA as adjuvant

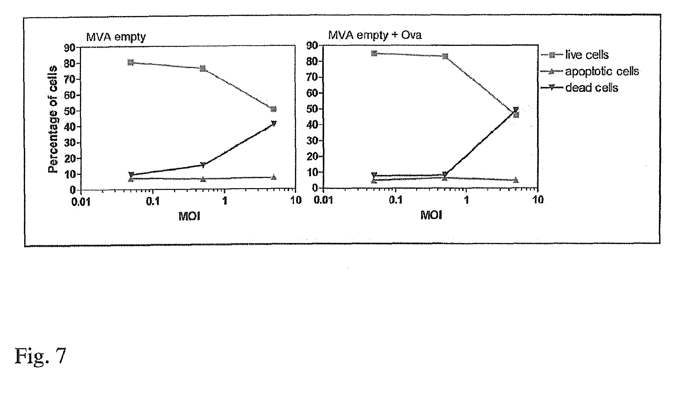

The obtained results demonstrated that the addition of the model antigen Ova does not alter the previously observed pattern of cell viability (Fig. 7). The number of live cells was higher at low (MOI: 0.05-0.5) than at high (MOI: 5) infection doses. Approximately half of the infected DCs retained their viability at high MOI 5, whereas most of cells infected at low MOI (~80%) were viable.

Fig. 7: Effect of MVA as adjuvant on the viability of infected DC in the presence or absence of Ova. Primary cultures of DCs were prepared as described, and an analysis of the viability of cells was performed after infection with MVA. The number of apoptotic (Annexin V-positive) and dead (7-AAD-positive) cells was determined by FACS gating on CD11c positive cells.

### 2.1.2 Effect on DC activation and maturation using MVA as adjuvant

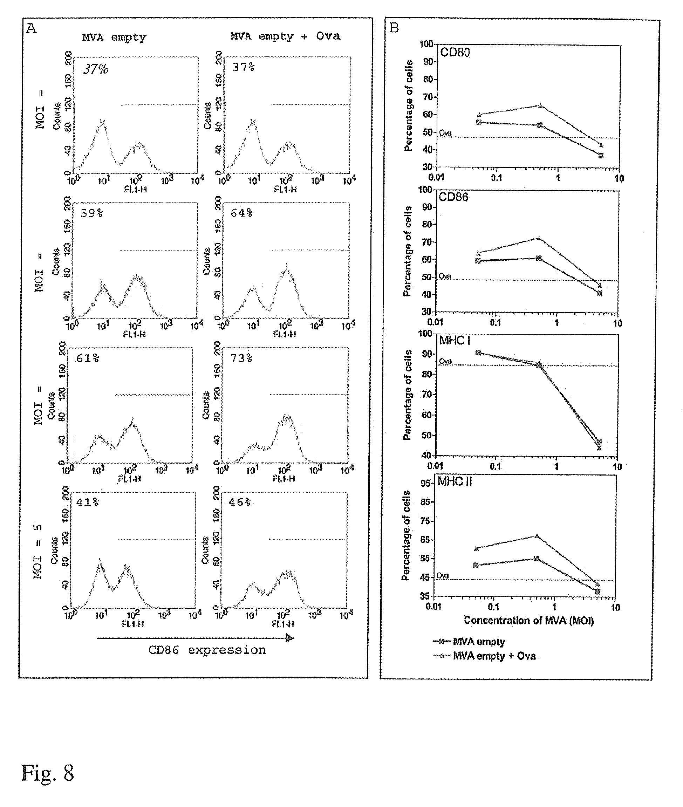

The effect of MVA on the DC activation was evaluated as described before (see 1.2). Two different populations of DCs could be distinguished by the expression levels of CD86 (*i.e.,* CD86^{lo} and CD86^{hi}) by FACS (Fig. 8, A). The proportions of the two sub-populations changed during infection with different concentrations of MVA. In uninfected DCs, the number of CD86^{lo} (non-activated, 63%) cells was higher than the number of CD86^{hi} (activated; ~37%) cells. The DC stimulation of Ova alone resulted in a slight increment of the activated population (~49%). When cells were infected at a MOI of 0.05 or 0.5, either with MVA alone or in combination with Ova, the number of activated DCs significantly increases (59-73%). In contrast, the number of activated cells was lower in DCs infected with MVA at MOI 5 (41-46%). A similar pattern was observed for the other tested activation and maturation markers (MHC I, MHC II, and CD80; Fig. 8, B).

Fig. 8: Expression of activation and maturation makers by DCs infected with MVA in the presence or absence of the model antigen Ova. Cells labelled with FITC conjugated antibodies against CD80, CD86, MHC class I and MHC class II were analysed by FACS, gating on CD11c⁺ cells. (A) Histograms of CD86 expression on DCs after infection in absence or presence of Ova at different MOIs. (B) Analysis of the expression markers on the DC surface at different MOIs in comparison to the expression of DCs incubated with Ova alone.

However, the strongest reduction was observed for MHC class I molecules, which were only expressed by ~40% of the cells infected at a MOI of 5. In contrast, when MVA was given at a MOI of 0.05, approximately 90% of the cells were positive. The expression of CD40 was also induced in cells infected at low MOI (data not shown). The presence of Ova did not affect the observed expression patterns.

### 2.1.3 Processing and presentation of soluble antigens to CD4⁺ T cells by DCs infected in vitro with empty MVA

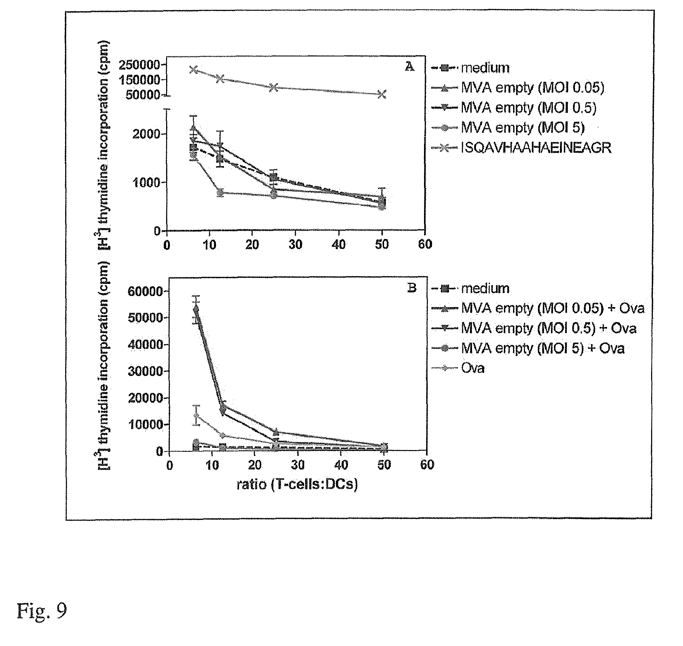

The capacity of MVA to improve the ability of DCs to process and present soluble Ova to antigen-specific CD4⁺ T cells was evaluated. To this end, T cells from OT-II transgenic mice, which express a TCR recognizing an epitope from Ova (ISQAVHAAHAEINEAGR) in the context of the MHC class II molecule I-A^{b}, were incubated with uninfected and MVA-infected DCs in the presence of Ova. The antigen-specific activation of CD4⁺ T cells was assessed by determining their proliferation capacity. As expected, MVA-infected DCs incubated in the absence of Ova were not able to induce proliferation of CD4⁺ T cells from OT-II mice, whereas efficient stimulation was observed when uninfected DCs were loaded with the Ova-peptide ISQAVHAAHAEINEAGR. More efficient stimulation of CD4⁺ T cells in response to Ova was observed when DCs were infected with MVA at low MOI (*i.e.,* 0.05 and 0.5) than using uninfected DCs or infected at MOI 5 (Fig. 9). This demonstrates that MVA exerts adjuvant activities *in vitro* and that this effect is only efficiently observed under the experimental conditions matter of this invention.

Fig. 9: Proliferation of T cells from OT-II mice after incubation with uninfected or MVA-infected DCs in absence (A) or presence (B) of Ova. Either DCs infected with the peptide (ISQAVHAAHAEINEAGR) or with the protein (Ova) were used as controls.

### 2.1.4 Processing and presentation of soluble antigens to CD8⁺ T cells by DCs infected in vitro with empty MVA

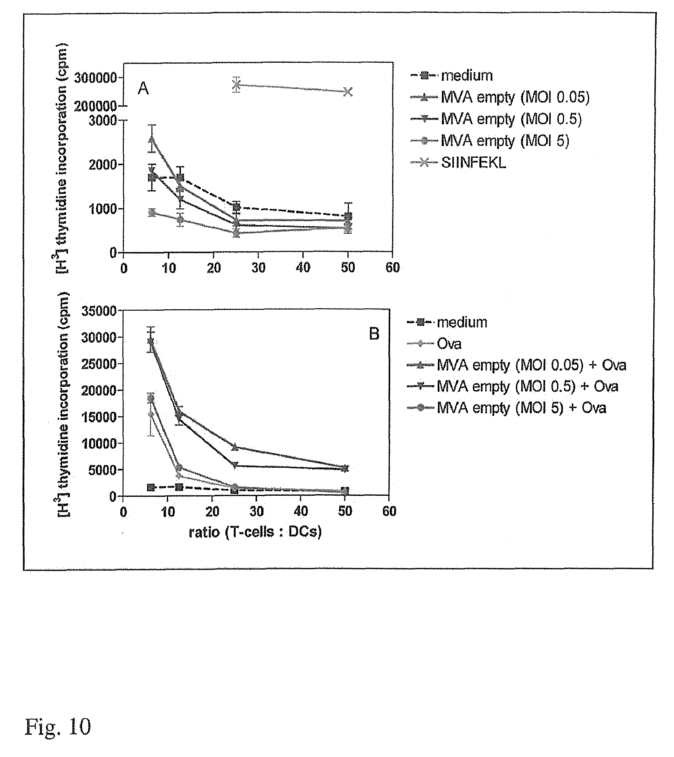

A similar approach was used to evaluate the capacity of MVA to improve the ability of DCs to process and present soluble Ova to antigen-specific CD8⁺ T cells. T cells from OT-1 transgenic mice, which express a TCR recognizing an epitope from Ova (SIINFEKL) in the context of MHC class I molecule (H-2k^{b}), were incubated with uninfected and MVA-infected DCs in the presence of Ova. The antigen-specific activation of CD8⁺ T cells was assessed by determining their proliferation capacity. As expected, uninfected and MVA-infected DCs incubated in the absence of Ova were not able to induce proliferation of cells from OT-I mice, whereas efficient stimulation was observed when uninfected DCs were loaded with the Ova-peptide SIINFEKL. More efficient stimulation of CD8⁺ T cells in response to Ova was observed when DCs were infected with MVA at low MOI (*i.e.,* 0.05 and 0.5) than using uninfected DCs or DCs infected at a MOI of 5 (Fig. 10). This confirms the immune modulatory properties of MVA, using the experimental conditions matter of this invention, and suggests that its use as adjuvant may lead to the improvement of both MHC class I and II-restricted responses.

Fig. 10: Proliferation of T cells from OT-I mice after incubation with uninfected or MVA-infected DCs in absence (A) or presence (B) of Ova. Either DCs infected with the peptide (SIINFEKL) or with the protein (Ova) were used as controls.

### 2.2 In vivo evaluation of MVA as immunomodulator

The strong immunomodulatory properties exhibited by MVA *in vitro* lead to evaluate its potential as adjuvant *in vivo.* To this end, mice were immunised by intramuscular route with Ova alone or add-mixed with empty MVA on day 0 and 28, and the immune responses elicited were evaluated.

**Table 1 Experimental groups**

| ***Experimental groups*** |
|---|
| • Control: PBS |
| • Ova (50 µg) |
| • MVA empty (10⁸ p.f.u.) |
| • MVA empty (10⁸ p.f.u.) + Ova (50 µg) |

### 2.2.1 No signs of acute toxicity were observed in mice immunised using MVA as adjuvant

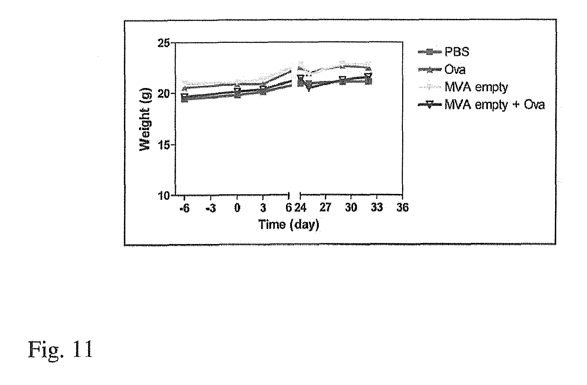

The overall safety of the vaccine formulations was assessed by monitoring different parameters including: animal weight (Fig. 11), food intake and general behavior during the course of immunisation, as well as by the examination of tissues and organs at sacrifice. No signs of acute toxicity (e.g., loss of weight) were observed in any of the experimental groups, thereby confirming that MVA is well-tolerated.

Fig.11: Weights of immunised mice (means per group).

### 2.2.2. Evaluation of the humoral immune responses obtained using MVA as adjuvant

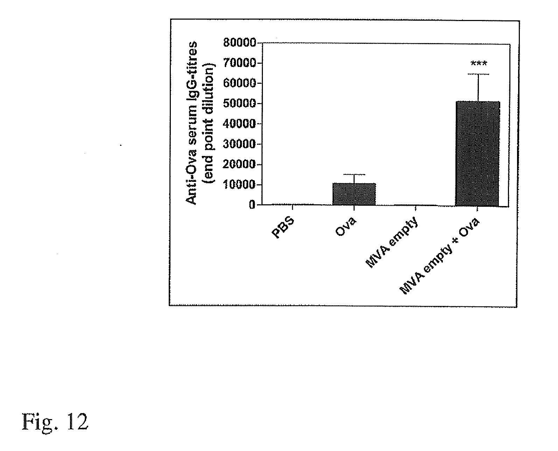

Antibodies represent a key effector mechanism in the defence against extracellular microbes and toxins [15]. The stimulation of Ova-specific IgG was determined in sera from vaccinated mice by ELISA. Mice immunised with either PBS or empty MVA did not produce antibodies against Ova (Fig. 12). On the other hand, immunisation with Ova alone or in combination with MVA empty resulted in the production of Ova-specific IgG. However, weak responses were observed in animals that received Ova alone, whereas those in which Ova was co-administered with MVA showed significantly higher antibody titres (p<0.001). This demonstrates the potential of MVA as adjuvant when antibody responses are needed.

### Fig. 12: Production of anti-Ova serum IgG in mice vaccinated using MVA as adjuvant.

Each bar represents the group mean end-point titre of anti-Ova IgG. The standard error of the mean is indicated by vertical lines. The results were statistically significant when compared with ANOVA at p<0.001 (***).

### Evaluation of specific IgG isotype titres

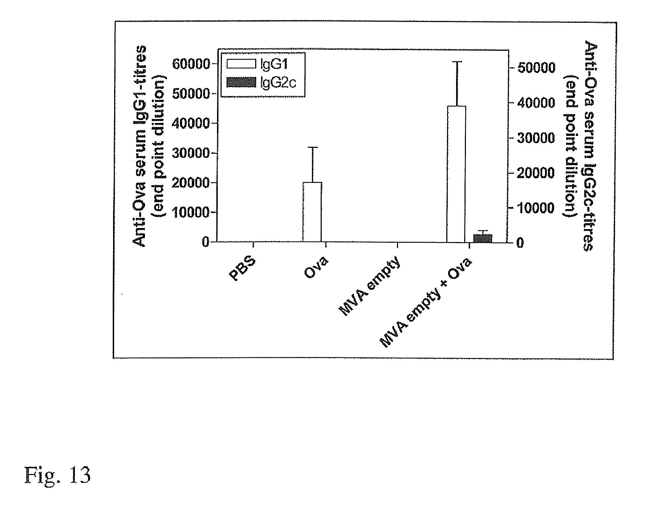

Antibodies can be divided into different isotypes, based on structural differences in their heavy chain regions (*i.e.*, IgM, IgG, IgA, IgD and IgE). The isotypes IgA and IgG can be further divided into subclasses. In mice the IgG isotype is divided into IgG1, IgG2a (or IgG2a-subtype b, also called IgG2c), IgG2b and IgG3. Titres of two main subtypes of IgG, namely IgG1 and IgG2c, were determined to differentiate the T_{H} pattern induced. High titres of IgG1 correlate with a T_{H}2-dominant response, whereas IgG2a (IgG2c in C57BL/6) is the main marker of a T_{H}1 response. Vaccination with Ova alone or co-administered with empty MVA resulted in an enhanced production of IgG1 (Fig. 13). Thus, the observed IgG subclasses correspond to a dominant T_{H}2-like response pattern. This is in contrast to the results obtained using recombinant MVA as delivery system, which are characterised by the stimulation of T_{H}1-based responses.

Fig. 13: Anti-Ova serum IgG1/IgG2c-titres obtained in mice vaccinated using MVA as adjuvant. Each bar represents the group mean end-point titre. The standard error of the mean is indicated by vertical lines.

### 2.2.3 Evaluation of the cellular responses stimulated using MVA as adjuvant

### Determination of IL-2- and IL-4-secreting cells stimulated after vaccination

The numbers of IL-2- and IL-4-secreting cells in spleens of immunised mice were determined by ELISPOT. IL-2 is responsible for T cell clonal expansion after antigen recognition and leads to an increased production of IL-4 and IFN-γ. IL-4 stimulates the development of T_{H}2 cells from naive CD4⁺ T cells, acting as an autocrine growth factor. It also regulates IgG class switching to IgG1 and IgE in mice inhibiting the switch to IgG2c and IgG3. This cytokine also antagonises the macrophage-activating effects of IFN-γ and inhibits cellular immune reactions [15].

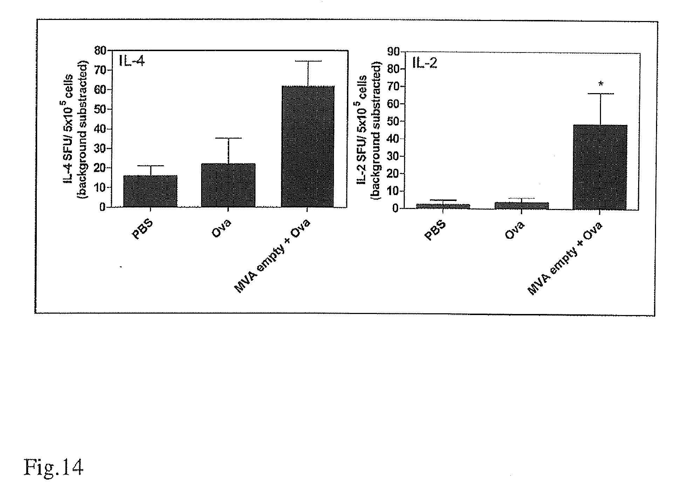

Co-administration of empty MVA with Ova induced the highest observed numbers of IL-2-secreting cells (p<0.05; Fig. 14 right panel). Low number of IL-2-producing cells were obtained in the control groups. The number of IL-4-producing cells was increased in mice immunised with MVA add-mixed with Ova. This correlates with the observed increase in the production of Ova-specific IgG1 (Fig. 13).

Fig. 14: Numbers of IL-2- and IL-4-producing cells in mice vaccinated using MVA as adjuvant. Cells were re-stimulated with Ova protein. Results are presented as specific IL-4-(left panel) and IL-2-spot forming units (SFU)/5 x 10⁵ cells (right panel). The standard error of the mean of quadruplicate values is indicated by vertical lines. The values reported are those obtained from stimulated cells subtracted of background from unstimulated cells. Unstimulated cells were lower than 30 SFU/5 x 10⁵ cells. The results were statistically significant when compared with ANOVA at p<0.05 (*).

### Determination of IFN-γ-secreting CD8⁺ T cells stimulated after vaccination

The numbers of IFN-γ-secreting cells in spleens of immunised mice were determined by ELISPOT. IFN-γ is secreted by T_{H}1 cells, which promotes their own development by stimulating the production of IL-12 by macrophages and inhibits the proliferation of T_{H}2 cells. T_{H}1 cells also provide helper signals to CD8⁺ CTLs, and non-specific killer cells, including NK cells and macrophages. When MHC class I restricted peptides are known, they can be used to determine the number of IFN-γ-secreting cells in spleens, which correlates with the cytotoxic response mediated by CD8⁺ T cells.

The determination of the number of specific IFN-γ secreting CD8⁺ T cells was performed by ELISPOT using three different types of peptides (*i.e.*, dominant, subdominant and cryptic) (Table 2)

**Table 2: Ova-peptides used for ELISPOT**

| **Amino acids** | **Sequence** | **Hierarchy** | **References** |
|---|---|---|---|
| **257-264** | SIINFEKL | Dominant | [78,85] |
| **55-62** | KVVRFDKL | Subdominant | [77,86] |
| **11-18** | CFDVFKEL | Cryptic | [76] |

Co-administration of empty MVA with Ova resulted in a higher number of IFN-γ-secreting CD8⁺ T cells than using Ova alone (p<0.001) (Fig. 15). This demonstrated the potential of MVA as adjuvant not only to induce antibody production, but also for the stimulation of IFN-γ-producing CD8⁺ T cells. The use of MVA as adjuvant did not alter the hierarchy of the peptides presented by MHC class I molecule (data not shown).

Fig. 15: Numbers of IFN-γ-producing CD8⁺ cells in mice vaccinated using MVA as adjuvant. (A) Cells were re-stimulated with the dominant Ova-peptide SIINFEKL. Results are presented as specific IFN-γ spot forming units (SFU)/5 x 10⁵ cells. The standard error of the mean of quadruplicate values is indicated by vertical lines. The values reported are those obtained from stimulated cells subtracted of background from unstimulated cells. Unstimulated cells were lower than 30 IFN-γ- SFU/5 x 10⁵ cells. (B) Every spot is representing a single IFN-γ secreting cell. The results were statistically significant when compared with ANOVA at p<0.01 (**) and p<0.001 (***).

### In vivo determination of the cytotoxic CD8⁺ T mediated response stimulated after vaccination

Direct assessment of cytotoxicity *in vivo* can be achieved by monitoring the survival of injected fluorescent targets relatively to a differentially labelled internal control population. This *in vivo* assay was performed to detect the capacity of cytotoxic T cells in immunised mice to cause death of cells labelled with the specific epitope.

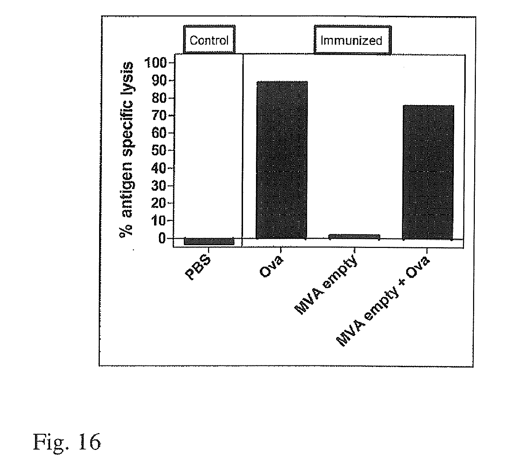

No cytotoxic activity was detected in mice immunised with either PBS or MVA empty. These results correlate with those obtained in the IFN-γ ELISPOT. In contrast, a high *in vivo* CTL response was obtained in mice vaccinated with MVA empty and Ova. However, high cytotoxic activity was also observed in mice immunised with Ova alone (Fig. 16). This can be explained by the late time point selected for the assay (40 h), which resulted in a saturation of the response. In future studies, an earlier time point will be selected to determine the CTL *in vivo.*

Fig. 16: in vivo analysis of specific lysis stimulated in mice vaccinated with MVA as adjuvant. Labelled control (CFSE^{lo}) and target cells pulsed with the Ova-peptide SIINFEKL (CFSE^{hi}) were transferred to vaccinated mice. After 40 h mice were sacrificed and in vivo lysis was analysed by FACS. Each bar represents the group mean for the % of specific lysis.

References:
[1] Murray, C. and Lopez, A. (2002) Reducing risks, promoting healthy life. pp. 186-197, World Health Organization, Genova.
[2] Mestecky, J., Bienenstock, J., McGhee, J.R., Lamm, M.E., Strober, W., Cebra, J.J., Mayer, L. and Ogra, P.L. (2005) Historical aspects of mucosal immunology. In: Mucosal Immunology (Mestecky, J. et al., eds.).
[3] Langer, W.L. (1976) Immunization against smallpox before Jenner. Sci Am 234, 112-7.
[4] Cruse, J.M. and Lewis, R.E. (2005) Historical Atlas of Immunology, Taylor & Francis, UK.
[5] Plotkin, S.A. (2005) Vaccines: past, present and future. Nat Med 11, S5-11.
[6] Janeway, C.A., Travers, P., Walport, M. and Shlomchik, M. (2001) Immunobiology, Garland Publishing, New York and London.
[7] Baxby, D., Ashton, D.G., Jones, D., Thomsett, L.R. and Denham, E.M. (1979) Cowpox virus infection in unusual hosts. Vet Rec 104, 175.
[8] Salmon, D.E. and Smith, T. (1886) On a new method of producing immunity from contagious diseases. Am Vet Rev 10, 63-69.
[9] Silverstein, A.M. (1999) Paul Ehrlich's passion: the origins of his receptor immunology. Cell Immunol 194,213-21.
[10] Glenny, A.T. and Hopkins, B.E. (1923) Diphtheria toxoid as an immunising agent. Br J Exp Pathol 4, 283-288.
[11] Bramwell, V.W. and Perrie, Y. (2005) The rational design of vaccines. Drug Discov Today 10, 1527-34.
[12] Bender, B.S., Rowe, C.A., Taylor, S.F., Wyatt, L.S., Moss, B. and Small, P.A., Jr. (1996) Oral immunization with a replication-deficient recombinant vaccinia virus protects mice against influenza. J Virol 70, 6418-24.
[13] Durbin, A.P. and Karron, R.A. (2003) Progress in the development of respiratory syncytial virus and parainfluenza virus vaccines. Clin Infect Dis 37, 1668-77.
[14] Lambert, P.H., Liu, M. and Siegrist, C.A. (2005) Can successful vaccines teach us how to induce efficient protective immune responses? Nat Med 11, S54-62.
[15] Abbas, A.K. and Lichtman, A.H. (2003) Cellular und molecular immunology, pp. 562, Saunders (W.B.) Co Ltd.
[16] Gasque, P. (2004) Complement: a unique innate immune sensor for danger signals. Mol Immunol 41, 1089-98.
[17] Janeway, C.A., Travers, P., Walport, M. and Shlomchik, M.J. (2005) Immunobiology, Garland Science Publishing.
[18] Carroll, M.C. and Prodeus, A.P. (1998) Linkages of innate and adaptive immunity. Curr Opin Immunol 10, 36-40.
[19] Levy, D.E., Marie, I. and Prakash, A. (2003) Ringing the interferon alarm: differential regulation of gene expression at the interface between innate and adaptive immunity. Curr Opin Immunol 15, 52-8.
[20] Ohteki, T. and Koyasu, S. (2001) Role of antigen-presenting cells in innate immune system. Arch Immunol Ther Exp (Warsz) 49 Suppl 1, S47-52.
[21] Reis e Sousa, C. (2004) Activation of dendritic cells: translating innate into adaptive immunity. Curr Opin Immunol 16, 21-5.
[22] Blach-Olszewska, Z. (2005) Innate immunity: cells, receptors, and signaling pathways. Arch Immunol Ther Exp (Warsz) 53, 245-53.
[23] Matzinger, P. (1998) An innate sense of danger. Semin Immunol 10, 399-415.
[24] Steinman, R.M., Inaba, K., Turley, S., Pierre, P. and Mellman, I. (1999) Antigen capture, processing, and presentation by dendritic cells: recent cell biological studies. Hum Immunol 60,562-7.
[25] Fonteneau, J.F., Larsson, M. and Bhardwaj, N. (2002) Interactions between dead cells and dendritic cells in the induction of antiviral CTL responses. Curr Opin Immunol 14, 471-7.
[26] Banchereau, J. and Steinman, R.M. (1998) Dendritic cells and the control of immunity. Nature 392, 245-52.
[27] Levin, D., Constant, S., Pasqualini, T., Flavell, R. and Bottomly, K. (1993) Role of dendritic cells in the priming of CD4+ T lymphocytes to peptide antigen in vivo. J Immunol 151, 6742-50.
[28] Yewdell, J.W., Anton, L.C. and Bennink, J.R. (1996) Defective ribosomal products (DRiPs): a major source of antigenic peptides for MHC class I molecules? J Immunol 157, 1823-6.
[29] Yewdell, J.W. (2001) Not such a dismal science: the economics of protein synthesis, folding, degradation and antigen processing. Trends Cell Biol 11, 294-7.
[30] Griffin, T.A., Nandi, D., Cruz, M., Fehling, H.J., Kaer, L.V., Monaco, J.J. and Colbert, R.A. (1998) Immunoproteasome assembly: cooperative incorporation of interferon gamma (IFN-gamma)-inducible subunits. J Exp Med 187, 97-104.
[31] Van den Eynde, B.J. and Morel, S. (2001) Differential processing of class-I-restricted epitopes by the standard proteasome and the immunoproteasome. Curr Opin Immunol 13, 147-53.
[32] Groettrup, M. and Schmidtke, G. (1999) [Intracellular processing of viral and tumor antigens by proteasomes]. Schweiz Med Wochenschr 129, 1660-5.
[33] Sijts, A.J., Ruppert, T., Rehermann, B., Schmidt, M., Koszinowski, U. and Kloetzel, P.M. (2000) Efficient generation of a hepatitis B virus cytotoxic T lymphocyte epitope requires the structural features of immunoproteasomes. J Exp Med 191, 503-14.
[34] Sijts, A.J., Standera, S., Toes, R.E., Ruppert, T., Beekman, N.J., van Veelen, P.A., Ossendorp, F.A., Melief, C.J. and Kloetzel, P.M. (2000) MHC class I antigen processing of an adenovirus CTL epitope is linked to the levels of immunoproteasomes in infected cells. J Immunol 164, 4500-6.
[35] Groettrup, M., van den Broek, M., Schwarz, K., Macagno, A., Khan, S., de Giuli, R. and Schmidtke, G. (2001) Structural plasticity of the proteasome and its function in antigen processing. Crit Rev Immunol 21, 339-58.
[36] Schumacher, R., Adamina, M., Zurbriggen, R., Bolli, M., Padovan, E., Zajac, P., Heberer, M. and Spagnoli, G.C. (2004) Influenza virosomes enhance class I restricted CTL induction through CD4+ T cell activation. Vaccine 22, 714-23.
[37] Cascio, P., Hilton, C., Kisselev, A.F., Rock, K.L. and Goldberg, A.L. (2001) 26S proteasomes and immunoproteasomes produce mainly N-extended versions of an antigenic peptide. Embo J 20, 2357-66.
[38] Gromme, M. and Neefjes, J. (2002) Antigen degradation or presentation by MHC class I molecules via classical and non-classical pathways. Mol Immunol 39, 181-202.
[39] Yewdell, J., Anton, L.C., Bacik, I., Schubert, U., Snyder, H.L. and Bennink, J.R. (1999) Generating MHC class I ligands from viral gene products. Immunol Rev 172, 97-108.
[40] Spellberg, B. and Edwards Jr., J.E. (2001) Type 1/Type 2 Immunity in Infectious Diseases. Clinical Infectious Diseases 32, 76-102.
[41] Hutchings, C.L., Gilbert, S.C., Hill, A.V. and Moore, A.C. (2005) Novel protein and poxvirus-based vaccine combinations for simultaneous induction of humoral and cell-mediated immunity. J Immunol 175, 599-606.
[42] Souza, A.P., Haut, L., Reyes-Sandoval, A. and Pinto, A.R. (2005) Recombinant viruses as vaccines against viral diseases. Braz J Med Biol Res 38, 509-22.
[43] Rocha, C.D., Caetano, B.C., Machado, A.V. and Bruna-Romero, O. (2004) Recombinant viruses as tools to induce protective cellular immunity against infectious diseases. Int Microbiol 7, 83-94.
[44] Molinier-Frenkel, V., Lengagne, R., Gaden, F., Hong, S.S., Choppin, J., Gahery-Segard, H., Boulanger, P. and Guillet, J.G. (2002) Adenovirus hexon protein is a potent adjuvant for activation of a cellular immune response. J Virol 76, 127-35.
[45] Moss, B. (1996) Poxviridae: the viruses and their replication (Fields of Virology, Volume 3rd Ed.), Lippincott-Raven, Philadelphia.
[46] Vanderplasschen, A. and Pastoret, P.P. (2003) The uses of poxviruses as vectors. Curr Gene Ther 3, 583-95.
[47] Mahalingam, S., Damon, I.K. and Lidbury, B.A. (2004) 25 years since the eradication of smallpox: why poxvirus research is still relevant. Trends Immunol 25, 636-9.
[48] Mayr, A. (2003) Smallpox vaccination and bioterrorism with pox viruses. Comp Immunol Microbiol Infect Dis 26, 423-30.
[49] Lane, J.M., Ruben, F.L., Abrutyn, E. and Millar, J.D. (1970) Deaths attributable to smallpox vaccination,1959 to 1966, and 1968. Jama 212, 441-444.
[50] Slifka, M.K. (2005) The Future of Smallpox Vaccination: is MVA the key? Med Immunol 4, 2.
[51] Belyakov, I.M., Earl, P., Dzutsev, A., Kuznetsov, V.A., Lemon, M., Wyatt, L.S., Snyder, J.T., Ahlers, J.D., Franchini, G., Moss, B. and Berzofsky, J.A. (2003) Shared modes of protection against poxvirus infection by attenuated and conventional smallpox vaccine viruses. Proc Natl Acad Sci U S A 100, 9458-63.
[52] McCurdy, L.H., Larkin, B.D., Martin, J.E. and Graham, B.S. (2004) Modified vaccinia Ankara: potential as an alternative smallpox vaccine. Clin Infect Dis 38, 1749-53.
[53] Meseda, C.A., Garcia, A.D., Kumar, A., Mayer, A.E., Manischewitz, J., King, L.R., Golding, H., Merchlinsky, M. and Weir, J.P. (2005) Enhanced immunogenicity and protective effect conferred by vaccination with combinations of modified vaccinia virus Ankara and licensed smallpox vaccine Dryvax in a mouse model. Virology 339, 164-75.
[54] Panicali, D. and Paoletti, E. (1992) Construction of poxviruses as cloning vectors: insertion of the thymidine kinase gene from herpes simplex virus into the DNA of infectious vaccinia virus. 1982. Biotechnology 24, 503-7.
[55] Mackett, M., Smith, G.L. and Moss, B. (1992) Vaccinia virus: a selectable eukaryotic cloning and expression vector. 1982. Biotechnology 24, 495-9.
[56] Erfle, V., Goebel, F.D., Guzman, C.A. and Le Grand, R. (2005) Vaccines based on Nef and on Nef/DeltaV2 Env. Microbes Infect 7, 1400-4.
[57] Drexler, I., Staib, C. and Sutter, G. (2004) Modified vaccinia virus Ankara as antigen delivery system: how can we best use its potential? Curr Opin Biotechnol 15, 506-12.
[58] Mayr, A., Hochstein-Mintzel, V. and Stickl, H. (1975) Passage history, properties, and applicability of the attenuated vaccinia virus strain MVA (in German). Infection 3, 6-14.
[59] Stickl, H., Hochstein-Mintzel, V., Mayr, A., Huber, H.C., Schafer, H. and Holzner, A. (1974) [MVA vaccination against smallpox: clinical tests with an attenuated live vaccinia virus strain (MVA) (author's transl)]. Dtsch Med Wochenschr 99, 2386-92.
[60] Hochstein-Mintzel, V., Huber, H.C. and Stickl, H. (1972) [Virulence and immunogenicity of a modified vaccinia virus (strain MVA) (author's transl)]. Z Immunitatsforsch Exp Klin Immunol 144, 104-56.
[61] Schroter, W., Stickl, H., Idel, H. and Schlipkoter, H.W. (1980) [MVA-vaccine in primovaccination against smallpox after the age of three (author's transl)]. Zentralbl Bakteriol Mikrobiol Hyg [B] 171, 309-19.
[62] Drexler, I., Heller, K., Wahren, B., Erfle, V. and Sutter, G. (1998) Highly attenuated modified vaccinia virus Ankara replicates in baby hamster kidney cells, a potential host for virus propagation, but not in various human transformed and primary cells. J Gen Virol 79 (Pt 2), 347-52.
[63] Staib, C., Drexler, I. and Sutter, G. (2004) Construction and isolation of recombinant MVA. Methods Mol Biol 269, 77-100.
[64] Moss, B. (1996) Stellungnahme der Zentralen Kommission für biologische Sicherheit; AZ 6790-10-14. In: FRG, 5/1997, Berlin.
[65] Antoine, G., Scheiflinger, F., Domer, F. and Falkner, F.G. (1998) The complete genomic sequence of the modified vaccinia Ankara strain: comparison with other orthopoxviruses. Virology 244, 365-96.
[66] Drexler, I., Antunes, E., Schmitz, M., Wolfel, T., Huber, C., Erfle, V., Rieber, P., Theobald, M. and Sutter, G. (1999) Modified vaccinia virus Ankara for delivery of human tyrosinase as melanoma-associated antigen: induction of tyrosinase- and melanoma-specific human leukocyte antigen A*0201-restricted cytotoxic T cells in vitro and in vivo. Cancer Res 59, 4955-63.
[67] Ramirez, J.C., Gherardi, M.M. and Esteban, M. (2000) Biology of attenuated modified vaccinia virus Ankara recombinant vector in mice: virus fate and activation of B- and T-cell immune responses in comparison with the Western Reserve strain and advantages as a vaccine. J Virol 74, 923-33.
[68] Staib, C., Lowel, M., Erfle, V. and Sutter, G. (2003) Improved host range selection for recombinant modified vaccinia virus Ankara. Biotechniques 34, 694-6, 698, 700.
[69] Ramirez, J.C., Gherardi, M.M., Rodriguez, D. and Esteban, M. (2000) Attenuated modified vaccinia virus Ankara can be used as an immunizing agent under conditions of preexisting immunity to the vector. J Virol 74, 7651-5.
[70] Belyakov, I.M., Moss, B., Strober, W. and Berzofsky, J.A. (1999) Mucosal vaccination overcomes the barrier to recombinant vaccinia immunization caused by preexisting poxvirus immunity. Proc Natl Acad Sci U S A 96, 4512-7.
[71] Moorthy, V.S., Pinder, M., Reece, W.H., Watkins, K., Atabani, S., Hannan, C., Bojang, K., McAdam, K.P., Schneider, J., Gilbert, S. and Hill, A.V. (2003) Safety and immunogenicity of DNA/modified vaccinia virus ankara malaria vaccination in African adults. J Infect Dis 188, 1239-44.
[72] Clarke, S.R., Barnden, M., Kurts, C., Carbone, F.R., Miller, J.F. and Heath, W.R. (2000) Characterization of the ovalbumin-specific TCR transgenic line OT-I: MHC elements for positive and negative selection. Immunol Cell Biol 78, 110-7.
[73] Kelly, J.M., Sterry, S.J., Cose, S., Turner, S.J., Fecondo, J., Rodda, S., Fink, P.J. and Carbone, F.R. (1993) Identification of conserved T cell receptor CDR3 residues contacting known exposed peptide side chains from a major histocompatibility complex class I-bound determinant. Eur J Immunol 23, 3318-26.
[74] Robertson, J.M., Jensen, P.E. and Evavold, B.D. (2000) DO11.10 and OT-11 T cells recognize a C-terminal ovalbumin 323-339 epitope. J Immunol 164, 4706-12.
[75] Barnden, M.J., Allison, J., Heath, W.R. and Carbone, F.R. (1998) Defective TCR expression in transgenic mice constructed using cDNA-based alpha- and beta-chain genes under the control of heterologous regulatory elements. Immunol Cell Biol 76, 34-40.
[76] Lipford, G.B., Hoffman, M., Wagner, H. and Heeg, K. (1993) Primary in vivo responses to ovalbumin. Probing the predictive value of the Kb binding motif. J Immunol 150, 1212-22.
[77] Mo, A.X., van Lelyveld, S.F., Craiu, A. and Rock, K.L. (2000) Sequences that flank subdominant and cryptic epitopes influence the proteolytic generation of MHC class I-presented peptides. J Immunol 164, 4003-10.
[78] Catipovic, B., Dal Porto, J., Mage, M., Johansen, T.E. and Schneck, J.P. (1992) Major histocompatibility complex conformational epitopes are peptide specific. J Exp Med 176, 1611-8.
[79] Sadegh-Nasseri, S. and McConnell, H.M. (1989) A kinetic intermediate in the reaction of an antigenic peptide and I-Ek. Nature 337, 274-6.
[80] Hermans, I.F., Silk, J.D., Yang, J., Palmowski, M.J., Gileadi, U., McCarthy, C., Salio, M., Ronchese, F. and Cerundolo, V. (2004) The VITAL assay: a versatile fluorometric technique for assessing CTL- and NKT-mediated cytotoxicity against multiple targets in vitro and in vivo. J Immunol Methods 285, 25-40.
[81] Engelmayer, J., Larsson, M., Subklewe, M., Chahroudi, A., Cox, W.I., Steinman, R.M. and Bhardwaj, N. (1999) Vaccinia virus inhibits the maturation of human dendritic cells: a novel mechanism of immune evasion. J Immunol 163, 6762-8.
[82] Behboudi, S., Moore, A., Gilbert, S.C., Nicoll, C.L. and Hill, A.V. (2004) Dendritic cells infected by recombinant modified vaccinia virus Ankara retain immunogenicity in vivo despite in vitro dysfunction. Vaccine 22, 4326-31.
[83] Sansom, D.M., Manzotti, C.N. and Zheng, Y. (2003) What's the difference between CD80 and CD86? Trends Immunol 24, 314-9.
[84] Schijns, V.E. (2000) Immunological concepts of vaccine adjuvant activity. Curr Opin Immunol 12, 456-63.
[85] Rotzschke, O., Falk, K., Stevanovic, S., Jung, G., Walden, P. and Rammensee, H.G. (1991) Exact prediction of a natural T cell epitope. Eur J Immunol 21, 2891-4.
[86] Niedermann, G., Butz, S., Ihlenfeldt, H.G., Grimm, R., Lucchiari, M., Hoschutzky, H., Jung, G., Maier, B. and Eichmann, K. (1995) Contribution of proteasome-mediated proteolysis to the hierarchy of epitopes presented by major histocompatibility complex class I molecules. Immunity 2, 289-99.
[87] Blanchard, T.J., Alcami, A., Andrea, P. and Smith, G.L. (1998) Modified vaccinia virus Ankara undergoes limited replication in human cells and lacks several immunomodulatory proteins: implications for use as a human vaccine. J Gen Virol 79 (Pt 5), 1159-67.
[88] Albert, M.L., Sauter, B. and Bhardwaj, N. (1998) Dendritic cells acquire antigen from apoptotic cells and induce class I-restricted CTLs. Nature 392, 86-9.
[89] Stuart, L.M., Lucas, M., Simpson, C., Lamb, J., Savill, J. and Lacy-Hulbert, A. (2002) Inhibitory effects of apoptotic cell ingestion upon endotoxin-driven myeloid dendritic cell maturation. J Immunol 168, 1627-35.
[90] Liu, K., Iyoda, T., Satemus, M., Kimura, Y., Inaba, K. and Steinman, R.M. (2002) Immune tolerance after delivery of dying cells to dendritic cells in situ. J Exp Med 196, 1091-7.
[91] Gallucci, S., Lolkema, M. and Matzinger, P. (1999) Natural adjuvants: endogenous activators of dendritic cells. Nat Med 5, 1249-55.
[92] Vogel, T.U., Horton, H., Fuller, D.H., Carter, D.K., Vielhuber, K., O'Connor, D.H., Shipley, T., Fuller, J., Sutter, G., Erfle, V., Wilson, N., Picker, L.J. and Watkins, D.I. (2002) Differences between T cell epitopes recognized after immunization and after infection. J Immunol 169, 4511-21.
[93] Albert, M.L. (2001) Phagocytosis of apoptotic cells. In: Dendritic Cells (Lotze, M.L. and Thomson, A.W., eds.), pp. 627-644, Academic Press, UK.
[94] Rock, K.L., Hearn, A., Chen, C.J. and Shi, Y. (2005) Natural endogenous adjuvants. Springer Semin Immunopathol 26, 231-46.
[95] Cosma, A., Nagaraj, R., Buhler, S., Hinkula, J., Busch, D.H., Sutter, G., Goebel, F.D. and Erfle, V. (2003) Therapeutic vaccination with MVA-HIV-1 nef elicits Nef-specific T-helper cell responses in chronically HIV-1 infected individuals. Vaccine 22, 21-9.
[96] McConkey, S.J., Reece, W.H., Moorthy, V.S., Webster, D., Dunachie, S., Butcher, G., Vuola, J.M., Blanchard, T.J., Gothard, P., Watkins, K., Hannan, C.M., Everaere, S., Brown, K., Kester, K.E., Cummings, J., Williams, J., Heppner, D.G., Pathan, A., Flanagan, K., Arulanantham, N., Roberts, M.T., Roy, M., Smith, G.L., Schneider, J., Peto, T., Sinden, R.E., Gilbert, S.C. and Hill, A.V. (2003) Enhanced T-cell immunogenicity of plasmid DNA vaccines boosted by recombinant modified vaccinia virus Ankara in humans. Nat Med 9, 729-35.
[97] Hanke, T., McMichael, A.J., Mwau, M., Wee, E.G., Ceberej, I., Patel, S., Sutton, J., Tomlinson, M. and Samuel, R.V. (2002) Development of a DNA-MVA/HIVA vaccine for Kenya. Vaccine 20, 1995-8.
[98] Moss, B., Carroll, M.W., Wyatt, L.S., Bennink, J.R., Hirsch, V.M., Goldstein, S., Elkins, W.R., Fuerst, T.R., Lifson, J.D., Piatak, M., Restifo, N.P., Overwijk, W., Chamberlain, R., Rosenberg, S.A. and Sutter, G. (1996) Host range restricted, non-replicating vaccinia virus vectors as vaccine candidates. Adv Exp Med Biol 397, 7-13.
[99] Abaitua, F., Rodriguez, J.R., Garzon, A., Rodriguez, D. and Esteban, M. (2005) Improving recombinant MVA immune responses: Potentiation of the immune responses to HIV-1 with MVA and DNA vectors expressing Env and the cytokines IL-12 and IFN-gamma. Virus Res.
[100] Makitalo, B., Lundholm, P., Hinkula, J., Nilsson, C., Karlen, K., Morner, A., Sutter, G., Erfle, V., Heeney, J.L., Wahren, B., Biberfeld, G. and Thorstensson, R. (2004) Enhanced cellular immunity and systemic control of SHIV infection by combined parenteral and mucosal administration of a DNA prime MVA boost vaccine regimen. J Gen Virol 85, 2407-19.
[101] Hodge, J.W., Poole, D.J., Aarts, W.M., Gomez Yafal, A., Gritz, L. and Schlom, J. (2003) Modified vaccinia virus ankara recombinants are as potent as vaccinia recombinants in diversified prime and boost vaccine regimens to elicit therapeutic antitumor responses. Cancer Res 63, 7942-9.
[102] Gherardi, M.M., Perez-Jimenez, E., Najera, J.L. and Esteban, M. (2004) Induction of HIV immunity in the genital tract after intranasal delivery of a MVA vector: enhanced immunogenicity after DNA prime-modified vaccinia virus Ankara boost immunization schedule. J Immunol 172, 6209-20.
[103] Mastrangelo, M.J., Eisenlohr, L.C., Gomella, L. and Lattime, E.C. (2000) Poxvirus vectors: orphaned and underappreciated. J Clin Invest 105, 1031-4.
[104] Moss, B. (1996) Genetically engineered poxviruses for recombinant gene expression, vaccination, and safety. Proc Natl Acad Sci U S A 93, 11341-8.
[105] Patel, D.D., Ray, C.A., Drucker, R.P. and Pickup, D.J. (1988) A poxvirus-derived vector that directs high levels of expression of cloned genes in mammalian cells. Proc Natl Acad Sci U S A 85, 9431-5.
[106] Davison, A.J. and Moss, B. (1990) New vaccinia virus recombination plasmids incorporating a synthetic late promoter for high level expression of foreign proteins. Nucleic Acids Res 18, 4285-6.
[107] Nelson, D., Bundell, C. and Robinson, B. (2000) In vivo cross-presentation of a soluble protein antigen: kinetics, distribution, and generation of effector CTL recognizing dominant and subdominant epitopes. J Immunol 165, 6123-32.
[108] Yewdell, J.W. and Bennink, J.R. (1999) Immunodominance in major histocompatibility complex class I-restricted T lymphocyte responses. Annu Rev Immunol 17, 51-88.
[109] Sercarz, E.E., Lehmann, P.V., Ametani, A., Benichou, G., Miller, A. and Moudgil, K. (1993) Dominance and crypticity of T cell antigenic determinants. Annu Rev Immunol 11, 729-66.
[110] Sandberg, J.K., Grufman, P., Wolpert, E.Z., Franksson, L., Chambers, B.J. and Karre, K. (1998) Superdominance among immunodominant H-2Kb-restricted epitopes and reversal by dendritic cell-mediated antigen delivery. J Immunol 160, 3163-9.
[111] van der Most, R.G., Sette, A., Oseroff, C., Alexander, J., Murali-Krishna, K., Lau, L.L., Southwood, S., Sidney, J., Chesnut, R.W., Matloubian, M. and Ahmed, R. (1996) Analysis of cytotoxic T cell responses to dominant and subdominant epitopes during acute and chronic lymphocytic choriomeningitis virus infection. J Immunol 157, 5543-54.
[112] Vitiello, A., Yuan, L., Chesnut, R.W., Sidney, J., Southwood, S., Farness, P., Jackson, M.R., Peterson, P.A. and Sette, A. (1996) Immunodominance analysis of CTL responses to influenza PR8 virus reveals two new dominant and subdominant Kb-restricted epitopes. J Immunol 157, 5555-62.
[113] Balter, M. (1998) Modest Briton stirs up storm with views on role of CTLs. Science 280, 1860-1.

## Claims

1. A method for *ex vivo* stimulation of antigen presenting cells (APC) comprising the steps of:
a) providing APC's and a composition containing one or more antigens and modified vaccinia virus Ankara (MVA) as an adjuvant;
b) infecting the APC's with the composition such that they are infected by MVA at a low MOI, thereby activating and maturing the APC's; and
c) isolating the activated/mature APC's.

2. The method of claim 1, wherein the MOI is between 0.02 and 0.5, preferably 0.05.

3. The method of claim 1 or 2, wherein the APC is selected from dendritic cells, activated B cells, monocytes, macrophages, activated T cells, hematological malignancies with antigen presenting capacities, EBV-transformed lymphoblastoid cell lines and/or lymph or vascular endothelial cells.

4. The method of one or more of claims 1-3, wherein the antigen is derived from the group consisting of therapeutic or prophylactic polypeptides, glycoproteins, lipoproteins, or polysaccharides and polypeptides, glycoproteins, lipoproteins, or polysaccharides of pathogenic agents, and functional parts thereof.

5. The method of claim 4, wherein the therapeutic polypeptide is derived from the group consisting of secreted proteins, e.g. polypeptides of antibodies, chemokines, cytokines or interferons.

6. The method of claim 4, wherein the pathogenic agent is derived from the group consisting of viruses, bacteria, protozoa, fungi and parasites as well as tumor cells or tumor cell associated antigens and functional parts thereof.

7. The method of claim 6, wherein the viruses are selected from the group consisting of influenza viruses, measles and respiratory syncytial viruses, dengue viruses, human immunodeficiency viruses, human hepatitis viruses, herpes viruses, or papilloma viruses.

8. The method of claim 6, wherein the protozoa is Plasmodium falciparum.

9. The method of claim 6, wherein the bacteria is tuberculosis-causing Mycobacteria or Bordetella pertussis.

10. The method of claim 6, wherein the tumor cell associated antigen is selected from the group consisting of melanoma-associated differentiation antigens, e.g. tyrosinase, tyrosinase-related proteins 1 and 2, of cancer testes antigens, e.g. MAGE-1,-2,-3, and BAGE, and of non-mutated shared antigens overexpressed on tumors, e.g. Her-2/neu, MUC-1, and p53.

11. The method of one or more of the preceding claims, wherein the MVA is non-recombinant.

12. A pharmaceutical composition containing the activated/mature APC's as isolated in one or more of claims 1-11 and a pharmaceutically acceptable carrier and/or diluent.

13. The pharmaceutical composition of claim 12, which is a vaccine.

14. The pharmaceutical composition of claim 12 or 13, which additionally comprises other compounds with immunomodulatory properties, preferably cytokines, chemokines, adjuvants/ immunomodulators, e.g., TLR-agonists such as MALP-2, poly IC, CpG, LPS, MPL.

15. Use of MVA as an adjuvant for the stimulation of APC *ex vivo*.

16. The use of claim 15, wherein the MVA is non-recombinant.

17. Use of the pharmaceutical composition of claims 12-14 in the manufacture of a medicament for the treatment of infectious diseases, chronic inflammatory or degenerative diseases, autoimmunity diseases, fungal diseases, cancer and allergies.


SEQUENCE LISTING

<110> Helmholtz-Zentrum für Infektionsforschung
      GSF-Forschungszentrum für Umwelt und Gesundheit GmbH

<120> Use of MVA as adjuvant in the stimulation of APC's

<130> P20881

<140> EP06121795.6
<141> 2006-10-05

<160> 4

<170> PatentIn version 3.3

<210> 1
<211> 17
<212> PRT
<213> Artificial

<220>
<223> Peptide derived from OVA

<400> 1

Ile Ser Gln Ala Val His Ala Ala His Ala Glu Ile Asn Glu Ala Gly
1               5                   10                  15

Arg

<210> 2
<211> 8
<212> PRT
<213> Artificial

<220>
<223> Peptide derived from OVA

<400> 2

Ser Ile Ile Asn Phe Glu Lys Leu
1               5

<210> 3
<211> 8
<212> PRT
<213> Artificial

<220>
<223> Peptide derived from OVA

<400> 3

Lys Val Val Arg Phe Asp Lys Leu
1               5

<210> 4
<211> 8
<212> PRT
<213> Artificial

<220>
<223>  Peptide derived from OVA

<400>  4

Cys Phe Asp Val Phe Lys Glu Leu
1               5